# EUROPEAN PATENT APPLICATION

(11) **EP 3 919 473 A1**
(43) Date of publication of application: **08.12.2021**
(21) Application number: 19912935.4
(22) Date of filing: 31.01.2019
(51) Int. Cl.: C07C 229/76, A23K 20/142, A23K 20/20

(54) **METHOD OF PRODUCING A COMPLEX MICRONUTRIENT FEED ADDITIVE BASED ON ORGANIC COMPOUNDS OF IRON, MANGANESE, ZINC, COPPER AND COBALT**

(71) Applicant: Aktsionernoe Obschestvo "Bioamid", Saratov, 410033 (RU)
(72) Inventor: VORONIN, Sergey Petrovich, 41004, g. Saratov (RU); GUMENIUK, Anatoliy Petrovich, Saratov, 410005 (RU); SINOLITSKY, Maksim Konstantinovich, Saratov, 410054 (RU)
(74) Representative: Berggren Oy
(86) International application number: PCT/RU2019/000059
(87) International publication number: WO 2020/159399

(57) **Abstract**

The invention relates to the field of chemical industry, in particular, to synthesis of chemical compounds, and relates to improving the method for producing biologically active forms of mixtures of complex compounds of essential microelements with aspartic acid for use as feed supplements for animals, in particular for farm animals. The method of obtaining a composition of complex compounds of microelements of iron (II), manganese (II), zinc (II), copper (II), and cobalt (II) with L-aspartic acid in a molar ratio of the corresponding metal and aspartic acid 1:2 and ammonium sulfate for the use as supplement in the animal feed includes the stage of sequential interaction of the monoammonium salt of L-aspartic acid with zinc, copper, cobalt, iron, and manganese sulfates in aqueous solution and the stage of water removal.

## Description

### Field of the invention

The invention relates to the field of chemical industry, in particular, to the synthesis of chemical compounds, and relates to improving the method for producing biologically active forms of compositions of complex compounds of essential microelements with aspartic acid for use as supplement in the animal feed, in particular for farm animals.

### Background of the invention

There is a known method for producing amino acid chelate compounds and their use (RU2567057). The method includes mechanical activating the metal oxides and/or metal carbonates and/or metal sulfates and/or metal chlorides and/or metal hydroxides in solid form and then converting them to amino acid chelate compounds in a solid-phase reaction. The metals from the list are used - copper, zinc, manganese, iron, magnesium, calcium, nickel, and cobalt. The mass ratio of metal compounds to amino acids is from 1:2 to 1:5. The method applies to amino acids - glycine, methionine, lysine or other amino acids or mixtures thereof. The reaction takes place under the influence of mechanical stress, shock and pressure in a finely chopping installation. When implementing the method, sticking of the product on the installation mechanisms is possible. This is especially characteristic of metal compounds with L-aspartic acid. The reaction does not always go right to the end and continues during product storage.

There is described the synthesis and properties of complex salts of biogenic acids of alkaline, alkaline earth and divalent metals (see Kadyrova R.G., Kabirov G.F., Mullakhmetov R.R., "Synthesis and properties of complex salts of biogenic acids of alkaline, alkaline earth and divalent 3d- Metals", Monograph, Kazan, 2016 - 169 p). There is presented the laboratory processes for synthesis of individual chelate complexes of lithium, calcium, magnesium, iron, manganese, zinc, copper, cobalt with glycine, methionine, aspartic acid, and glutamic acid. In particular, the following conditions are described for aspartic acid: adding sodium hydroxide (0.11 mol) to an acid suspension (0.05 mol), heating to 65 °C and adding metal sulfate in portions, cooling after 35 minutes and filtering off the precipitate formed, washing with an organic solvent and drying. The synthesis is characterized by a high pH value (about 12), at which, under conditions of increased temperature, side processes of hydrolysis of metal salts and oxidation of iron, manganese and cobalt can occur. In addition, an organic solvent is used. In the course of synthesis, chelate complexes of a complexing agent - metal - 1:1 composition, that are poorly soluble in water are obtained.

The patent US6458981 is known wherein chelating compounds of amino acids with metals (oxidation state + 2 and + 3) are obtained by mixing calcium oxide (hydroxide), amino acid and metal sulfate without heating in any order. Wherein, while stirring, a precipitate of calcium sulfate forms, that is either filtered off or left in the mixture and together with the dissolved substances, is fed to spray drying. The method is characterized by the presence of a complex stage of filtration, that can lead to losses of the target product, which the authors suggest avoiding by leaving a ballast precipitate of calcium sulfate mixed with the product. In cases where calcium sulfate is separated, calcium sulfate accumulates and needs to be disposed of.

The patent US6518240 is known wherein chelating compounds of amino acids with metals are obtained by mixing hydrated metal sulfates (individually or in a mixture) and amino acids (individually or in a mixture) in a dry state, and in a sealed plastic bag, the mixture is heated in an oven at a temperature of 50 to 100 °C for from 15 minutes to a day, then cooled and kept in a bag from several hours to a week to complete the process. A powdery product is obtained. As a result of the implementation of the method, a heterogeneous product is obtained (it is almost impossible to obtain a uniform distribution for the needed contacts for particles of the same size, different in nature), the solid particles of the reacting substances are in contact with a small limited surface, that does not contribute to the completeness of the reaction in hydrated water vapor.

There is known the patent CN104619194, that describes the preparation of complexes of amino acids (glycine, methionine, lysine) with biogenic metals (iron, manganese, copper, zinc). To do that, solutions of the amino acids with each of the metal sulfates are separately prepared. In the next stage, the solutions are mixed and then served for drying. Powder of mixture of amino acid complexes with metals in molar ratio of 1:1 is obtained, that is proposed to be used in animals feeding. The method is used for three amino acids and is not used under the described conditions for amino acids with low solubility, for example, for aspartic acid.

### Summary of the invention

The object of this invention is to develop a method for producing a composition of complex compounds of microelements of iron, manganese, zinc, copper, and cobalt with high biological activity and simplify the process of obtaining a composition of microelements that can be used in a complex microelement supplement in animal feed.

The said object is solved by developing a method of obtaining the composition of complex compounds of microelements of iron (II), manganese (II), zinc (II), copper (II), and cobalt (II) with L-aspartic acid in a molar ratio of the corresponding metal and aspartic acid - 1:2 and ammonium sulfate, that includes the stage of sequential interaction in an aqueous solution of the monoammonium salt of L-aspartic acid with zinc, copper, cobalt, iron, and manganese sulfate and the stage of water removal.

In some embodiments, after the interaction of the monoammonium salt of L-aspartic acid with copper sulfate, carbon dioxide is passed through the solution until neutral pH is reached.

In some particular embodiments, carbon dioxide is passed through until pH of 6.5-7.5 is reached.

In some embodiments, water is removed by spray drying.

In some embodiments, L-aspartic acid in the form of monoammonium salt solution is obtained during the microbiological biotransformation of fumaric acid.

In some embodiments, metal sulfates are used in the form of hydrates.

The invention also relates to a composition.

As a result of implementation of the invention, the following technical results are achieved:
- Developing a method for producing a composition of complex compounds of microelements of iron, manganese, zinc, copper, and cobalt with high biological activity that can be used in a complex microelement supplement in animal feed.
- Providing a composition of complex compounds of microelements with high uniformity of composition; stability and possibility of use in a wide range of concentrations;
- Developing a method for producing a composition of complex compounds of microelements with ammonium sulfate, that ensures the preservation of the initial structure of complex compounds at the stage of water removal, and also improves the quality of the ready composition;
- Simplifying a process of producing a composition of complex compounds of microelements by reducing the stages of the process, reducing the time of the process and providing the possibility of scalability of the process;
- Developing a method of obtaining the composition of complex compounds of microelements with L-aspartic acid, that provides reduction in the cost of L-aspartic acid.

### Terms and definitions

The following terms and definitions are used in this document unless otherwise is explicitly indicated.

In the description of this invention, the terms "includes" and "including" are deemed as meaning "includes, among other things". These terms are not intended to be interpreted as "consists only of".

The term "supplement" means an ingredient, premix that can be used for inclusion into animal feed or to obtain feed compositions (for example, feed stuff).

Unless otherwise specified, the technical and scientific terms in this application have standard meanings, generally accepted in the scientific and technical literature.

### Detailed description of the invention

This invention is aimed at development of method for producing a composition of complex compounds of microelements of iron, manganese, zinc, copper, and cobalt with high biological activity and simplification the process of obtaining a composition of microelements that can be used in a complex microelement supplement in animal feed.

The claimed method comprises obtaining a composition of complex compounds of microelements iron (II), manganese (II), zinc (II), copper (II) and cobalt (II) with L-aspartic acid and ammonium sulfate by implementing the stages of: sequential interaction of monoammonium salt of L-aspartic acid with sulfate of zinc, copper, cobalt, iron, and manganese and water removal.

One of the factors affecting biological activity is the degree of oxidation of the metal included in the complex compound (chelate complex). Metals like manganese (+2), cobalt (+2), iron (+2) are easily oxidized in an alkaline medium to an oxidation state of +3, getting strong oxidizing properties in acidic and weakly acidic medium. This state of metals is not characteristic of animal organism. The solution monoammonium salt of aspartic acid used in the process of producing coordination complexes, in the particular case of chelate complexes, is alkaline.

To prevent oxidation of these metals when metal salts come into contact with such a solution of aspartic acid monoammonium salt, the reaction is carried out at the first stage with zinc sulfate, then with copper sulfate, then with cobalt sulfate, then with ferrous sulfate, and then with manganese sulfate, wherein the alkalinity of the solution decreases. The complexation reaction with copper sulfate earlier than with cobalt, iron, and manganese sulfates prevents possible oxidation of the latter by copper (+2) in the alkaline medium.

In the particular case of the invention embodiment, the first stage is a reaction with zinc sulfate, then with copper sulfate. Then, carbon dioxide is passed through the solution until the medium reaches pH close to neutral, in one embodiments till pH of 6.5-7.5. Thereafter out a complexation reaction with the remaining metals is carried out by adding cobalt sulfate, then iron sulfate, and then manganese sulfate.

It is known that aspartic acid is poorly soluble in water due to strong intermolecular bonds in the crystals of the compound. Wherein such bonds are partially preserved in the dissolved part, forming peculiar polymers with a degree of polymerization in appreciable concentrations from 2 to 5, what is registered chromatographically. The complexation of aspartic acid with divalent metals from such a solution (suspension) leads to unequal composition of complexes with different biological activity. The conversion of aspartic acid into a soluble monoammonium salt by the action of ammonia solution on a crystalline amino acid (phase transition absent in animal and plant cells) does not completely eliminate the described problem.

In the course of research, it was found that to obtain a composition of complex compounds of microelements, it is possible to use a solution of monoammonium salt of aspartic acid, obtained as an intermediate product in the microbiological process of production of aspartic acid by biotransformation of fumaric acid, bypassing the phase transition stage for aspartic acid, and such the solution comprises exclusively monomolecules of ammonium salt of aspartic acid. The complexes of metals with L-aspartic acid and ammonium sulfate obtained with the use of such the solution exhibit in dry form properties of non-crystalline substances. When evaporating the solution, crystals do not form, and the solution passes through a viscous state into a glassy mass. The later in the form of a powder is easily soluble in water, that simplifies assimilation by animal organisms.

When feeding animals with a complex microelement supplement (based on the composition of complex compounds of microelements), the best biological activity is shown in comparison with a similar product obtained in the process with a phase transition of aspartic acid even under the action of sodium hydroxide (see example No.1). Therefore, a solution of monoammonium salt of aspartic acid obtained in the process of biotransformation of fumaric acid was used to prepare complex compounds, in the particular case of chelate complexes with biogenic metals.

The use of monoammonium salt of aspartic acid allows to get a product with stable homogeneous properties in contrast to the use of commercial crystalline aspartic acid from different manufacturers. A concomitant effect of the use of such the solution of monoammonium salt of aspartic acid is a reduction in the cost in re-calculation of ammonium salt to aspartic acid.

The simplification of the process of obtaining a composition of complex compounds of microelements is achieved by limiting the stages to two by using a ready-made solution (with the needed salt concentration and pH) of the monoammonium salt of aspartic acid, in particular from the intermediate stage of microbiological production of aspartic acid. The process includes a stage of dissolving the metal sulfates: iron (II), manganese (II), zinc (II), copper (II), and cobalt (II) in the ready solution of the monoammonium salt of aspartic acid and a stage of water removal. In one of embodiments, the metal sulfates are hydrates and/or crystalline hydrates.

Slow processes of water removal from multicomponent mixtures (crystallization by evaporation or under influence of a different solvent) can lead to formation of crystals of different compositions from pure individual components to various co-crystals, that makes the product inhomogeneous. At slow concentration, new by-processes are possible. In the course of our studies, it was found that ammonium sulfate formed during the implementation of the method and included in the complex compounds and their composition, in the process of obtaining the composition helps drying the components of the composition, that stipulates the preservation of the original structure of complex compounds at the stage of water removal. Ammonium sulfate also accelerates process of dissolving, what positively affects the bioavailability of the composition, thereby improving the quality of the ready composition.

In particular embodiment, water is removed by spray drying. During spray drying, the dry product is obtained instantly, and the possibility of the above unwanted processes is further reduced.

During the implementation of the method, a homogeneous composition of complex compounds of microelements with high biological activity is obtained, wherein each part of the composition comprises the entire list of complexes of biogenic metals with aspartic acid and ammonium sulfate in the same ratio.

The high biological activity of the feed supplement allows its use in a complex microelement supplement in animal feed, in particular for mammals and birds, including farm animals.

Wherein the percentages of the components of the composition are not limited and can be any, depending on the task; for example, different ratios are used in supplements for various farm animals: cows, pigs, broilers or laying hens.

### The method is carried out as follows.

The method of obtaining the composition of complex compounds of iron, manganese, zinc, copper, and cobalt with L-aspartic acid can be combined with the production of biotechnological production of L-aspartic acid in order to use ready intermediate product - monoammonium salt solution for the implementation of method. Wherein the monoammonium salt of L-aspartic acid can be obtained from ammonium fumarate (diammonium salt of fumaric acid) by the action of the aspartase enzyme in the biocatalyst.
1. Preparation of biocatalyst to obtain monoammonium salt of L-aspartic acid.
   The biocatalyst is cells of a recombinant strain of bacteria *E. coli* VKPM B-11745, a producer of the aspartase enzyme, immobilized in a matrix of covalently cross-linked polyethyleneimine.
   The biomass of bacteria is obtained by deep aerobic fermentation of the specified strain in bioreactor within 24-28 hours. Cells are separated from the culture fluid, washed and immobilized. The obtained immobilized cells are crushed, dried and sieved to the required particle size.
2. Obtaining a solution of monoammonium salt of L-aspartic acid.
   A flow reactor is filled with biocatalyst, and aqueous solution of ammonium fumarate (fumaric acid diammonium salt) (245 g/l), previously thermostated at 25 °C, is pumped through the reactor at a predetermined speed.
   A solution of ammonium fumarate is obtained by neutralizing fumaric acid with aqueous ammonia to pH of 8.5 in yield of 99.5%.
   The solution coming out of the reactor comprises about 250 g/l of the monoammonium salt of L-aspartic acid. The reaction solution is collected and used at the stage of obtaining the complex of microelements of metals.
3. Obtaining a complex of microelements of metals of iron, manganese, zinc, copper, and cobalt with L-aspartic acid and ammonium sulfate.

The process of obtaining includes two stages. At the first stage, to solution of L-aspartic acid monoammonium salt, used without further processing and heated to a temperature of 50 ± 5 °C, with periods of 15-30 min metal sulfates are successively added with quantities, calculated to achieve molar ratios of metal to aspartic acid 1:2: firstly zinc sulfate, then copper sulfate, and carbon dioxide is passed through the solution until close to neutral medium; after that, without interrupting supply of carbon dioxide, with periods of 15-30 min, cobalt sulfate, then iron sulfate, and then manganese sulfate are added. At the second stage, the solution is transferred to a spray dryer. The drying process proceeds at 110-120 °C.

In the process of spray drying, particles of the product about 100-200 microns size are formed, which is optimal for further preparation of feed compositions. A significant advantage of this complex is the identity of the composition of microelements in each particle of the complex.

It should be understood that these and all the examples given in the application materials are not limiting and are given only to illustrate the present invention.

### Example 1. Obtaining a complex microelement supplement in feed for broilers based on the composition of complex compounds.

Solution of monoammonium salt of aspartic acid with concentration 223.2 g/L and pH 8.5-9.0 (400 L) is loaded to a reactor with a stirrer, heating system and gas supply system. The solution is heat to 50 °C, and ZnSO₄·7H₂O (28.4 kg) is added under stirring, after 15 minutes CuSO₄·5H₂O (15.8 kg) is added, carbon dioxide is passed to pH of 6.5-7.5, CoSO₄·7H₂O (0.214 kg) is added, after 15 minutes FeSO₄·7H₂O (9.74 kg) is added, after 15 minutes MnSO₄·5H₂O (23.6 kg) is added, and after 30 minutes solution is transferred to a spray dryer with a drying mode of 110-120 °C. Finely divided blue powder (150 - 155 kg) is obtained with concentration of metals (g/kg): iron - 11.6; manganese - 46.2; zinc - 38.5; copper - 24.0; cobalt - 0.274. Taking into account high biological activity of such the mixture, it is enough to introduce 0.5 kg of the mixture per a ton of the feed.

### Example 2. The main indicators of broilers growing using a complex microelement supplement in feed based on the composition of complex compounds of microelements obtained by different technologies.

The tests were carried out in the experimental farm VNITIP, Sergiev Posad, Russia. There were 30 broilers in each group.

| Group | Feeding characteristic |
|---|---|
| 1 (control) | Feed stuff for broilers with microelements constituting 7.5% of the prescribed composition in the form of complexes with L-aspartic acid obtained with phase transition through crystalline aspartic acid |
| 2 (experiment) | Feed stuff for broilers with microelements constituting 7.5% of the prescribed composition in the form of complexes with L-aspartic acid obtained without phase transition through monoammonium salt |
| 3 (experiment) | Feed stuff for broilers with microelements constituting 5% of the prescribed composition in the form of complexes with L-aspartic acid obtained without phase transition through monoammonium salt |

The results of the experiment are presented in the table below:

| Indicators | 1 group (control) | 2 group (experience) | 3 group (experience) |
|---|---|---|---|
| Live weight of daily chickens, g | 37 | 37 | 37 |
| Live weight of the bird in 37 days, g | 2013.4 | 2076.0 + 3,1% | 2062.3 + 2,4% |
| Feed costs per 1 kg of growth, kg | 1.67 | 1.59 | 1.65 |
| The average daily growth, g | 53.40 | 55.11 | 54.73 |

A mixture of microelements in organic form with L-aspartic acid obtained through the monoammonium salt of aspartic acid according to the method of this application shows (group 2) the best indicators for broilers growing (bird weight after 37 days of growing, feed costs per 1 kg of growth, average daily growth), that is an indicator of the biological activity of microelements.

## Claims

1. A method of obtaining the composition of complex compounds of microelements of iron (II), manganese (II), zinc (II), copper (II), and cobalt (II) with L-aspartic acid in a molar ratio of the corresponding metal and aspartic acid 1:2 and ammonium sulfate for use as a supplement in the animal feed, that includes the stage of sequential interaction of monoammonium salt of L-aspartic acid with zinc, copper, cobalt, iron, manganese sulfates in aqueous solution and the stage of water removal.

2. The method according to claim 1, wherein, after the interaction of the monoammonium salt of L-aspartic acid with copper sulfate, carbon dioxide is passed through the solution until neutral pH is reached.

3. The method according to claim 2, wherein passing carbon dioxide until pH of 6.5-7.5 is reached.

4. The method according to claim 1, wherein water is removed during the spray drying process.

5. The method according to claim 1, wherein L-aspartic acid is obtained in the form of a monoammonium salt solution during the microbiological biotransformation of fumaric acid.

6. The method according to claim 1, wherein metal sulfates are used in the form of hydrates.

7. A composition obtained by the method according to any one of claims 1 to 6.
